(19) Europäisches Patentamt
European Patent Office
Office européen des brevets

(11) Veröffentlichungsnummer: **0 230 250 B1**

# EUROPÄISCHE PATENTSCHRIFT

(45) Veröffentlichungstag der Patentschrift: **03.04.91**

(51) Int. Cl.[5]: **C07D 307/46**

(21) Anmeldenummer: **87100311.7**

(22) Anmeldetag: **13.01.87**

(54) **Verfahren zur Herstellung von 5-Hydroxymethylfurfural einschliesslich eines kristallinen Produktes unter ausschliesslicher Verwendung von Wasser als Lösungsmittel.**

(30) Priorität: **17.01.86 DE 3601281**

(43) Veröffentlichungstag der Anmeldung:
**29.07.87 Patentblatt 87/31**

(45) Bekanntmachung des Hinweises auf die Patenterteilung:
**03.04.91 Patentblatt 91/14**

(84) Benannte Vertragsstaaten:
**AT BE CH DE FR GB IT LI NL SE**

(56) Entgegenhaltungen:
**US-A- 2 929 823**

**JOURNAL OF CHROMATOGRAPHY, Band 322, Nr. 3, 10. April 1985, Seiten 411-424, Elsevier Science Publishers, Amsterdam, NL; G. BONN: "High-performance liquid chromatographic elution behaviour of oligosaccharides, monosaccharides and sugar degradation products on series-connected ion-exchange resin columns using water as the mobile phase"**

**Methods in Carbohydrate Chemistry, Vol. VI, P. 65-75, Vol. VIII P. 3-12**

(73) Patentinhaber: **SÜDZUCKER AKTIENGESELLSCHAFT MANNHEIM/OCHSENFURT**
**Maximilianstrasse 10**
**W-6800 Mannheim 1(DE)**

(72) Erfinder: **Rapp, Knut M., Dr. Dipl.-Chem.**
**Im Kerner 16**
**W-6521 Offstein(DE)**

(74) Vertreter: **Körber, Wolfhart, Dr.rer.nat. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**W-8000 München 22(DE)**

**EP 0 230 250 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Herstellung von 5-Hydroxymethlfurfural aus Sacchariden, wobie aus einer durch katalytische Zersetzung der Saccharide erhaltenen Reaktionslösung HMF unter ausschließlicher Verwendung von Wasser als Elutionsmittel über Ionenaustauscher abgetrennt wird.

Ein intramolekulares, dreifaches Dehydratisierungsprodukt von Hexosen ist 5-Hydroxymethylfurfural (im folgenden HMF genannt) mit der allgemeinen Formel

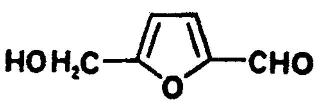

(I).

Nachwachsende Rohstoffe, wie Stärke, Cellulose, Saccharose oder Inulin, sind preiswerte Ausgangssubstanzen zur Herstellung von Hexosen, wie Glucose und Fructose.

HMF besitzt u. a. antibakterielle und korrosionsinhibierende Eigenschaften und ist für eine Vielzahl von Reaktionen geeignet. Es ist ohne große Schwierigkeiten möglich, daraus Furfuryldialkohol,-dialdehyd und -dicarbonsäure und deren Derivate herzustellen; desgleichen führt die Hydrierung des Ringes zu difunktionellen 2,5-Tetrahydrofuranderivaten. Auch an C-2 und C-5 unterschiedlich substituierte difunktionelle Furanderivate sind aus HMF gut zugänglich.

HMF ist im Prinzip eine Zwischenstufe der dehydratisierenden Zersetzung von Hexosen zur Levulinsäure und Ameisensäure, d. h. es kommt entscheidend darauf an, die Reaktion zum richtigen Zeitpunkt abzustoppen. Damit wird die Abtrennung von HMF von den Ausgangszuckern und Nebenprodukten zu einem wichtigen Schritt bei seiner Herstellung.

Als Ausgangsstoff für eine, auch im großen Maßstab durchführbare Herstellung von HMF kommen neben Fructose, insbesondere fructoseenthaltende Kohlenhydrate, Saccharose, Fructosesirupe, z. B. high fructose corn syrup (HFCS, Iso-glucose), Mutterlaugen der Fructosekristallisation oder polymere Fructoside, wie Inulin, in Frage. Selbst die Inulin enthaltende Zichorie (Inulingehalt ca. 18%) eignet sich direkt oder als mit Wasser gemischtes Mus für die Herstellung einer Fructose und HMF-enthaltenden Lösung. Da Glucose, auch in polymerer Form als Stärke bzw. Cellulose, durch Photosynthese in nahezu unbegrenzter Menge synthetisiert wird und eine Isomerisierung der Glucose in Fructose heute in größtem Maßstab durchführbar ist, sind Ausgangsstoffe zur Synthese von HMF in großen Mengen leicht zugänglich.

Als besonders geeigneter Ausgangsstoff für die Herstellung von HMF sind Mutterlaugen der Fructosekristallisation zu nennen.

Als Katalysator für die Dehydratisierung sind unterschiedliche Säuren bzw. Salze beschrieben worden, z. B. Oxalsäure (vgl. W. N. Haworth et al, J. Chem. Soc. 1944 , 667), Salze, wie Pyridinhydrochlorid (vgl. C. Fayet et al, Carbohydr. Res. 122 , 59 (1983)), saure Ionenaustauscher (vgl. DE-OS 30 33 527) oder Lewissäuren, wie Zirkonylchlorid (vgl. SU 1 054 349, zit. CA 100 , 120866s) oder Bortrifluorid-Etherat (vgl. H. H. Szmant et al, J. Chem. Tech. Biotechnol 31 , 135 (1981)).

Für die großtechnische HMF-Produktion sollte der eingesetzte Katalysator preiswert und unkorrosiv sein. Feste, zur Wiederverwendung bestimmte Katalysatoren sind wegen der leichten Bildungsweise von unlöslichen Nebenprodukten deshalb ungeeignet, weil eine Abtrennung des Katalysators (z. B. Ionenaustauscher) von diesen Nebenprodukten unwirtschaftlich bzw. unmöglich ist. Lewissäuren, wie Zirkonylchlorid oder Aluminiumchlorid, sind aus Korrosionsschutzüberlegungen ebenfalls abzulehnen. Als günstig wird deshalb der Einsatz von Schwefelsäure oder Phosphorsäure angesehen, da in diesem Fall die sauren wäßrigen Reaktionslösungen ggf. mit Basen neutralisiert werden können und etwa bei der Verwendung von Calciumhydroxyd oder Calciumcarbonat die Überführung der Katalysatorsäuren in schwer lösliche Salze mit einer Abfiltration möglich ist.

Das Reaktionsmedium der Dehydratisierung von Sacchariden wird durch ihre Löslichkeit bestimmt. Neben Wasser sind insbesondere dipolare, aprotische Lösungsmittel, wie Dimethylformamid oder Dimethylsulfoxid, verwendet worden.

Die jodkatalysierte Umwandlung des Fructoseteiles der Saccharose zu HMF durch Erhitzen von Saccharose in wasserfreiem Dimethylformamid erfordert neben dem teueren Lösungsmittel außerdem eine aufwendige Aufarbeitung, nämlich Extraktion und Papierchromatographie (vgl. T. G. Bonner et al, J. Chem. Soc. 1960 , 787).

In Dimethylsulfoxid werden bei der Zersetzung von Fructose mit unterschiedlichen Katalysatoren gute Ausbeuten (> 90%) an HMF gefunden (vgl. H. H. Szmant et al, J. Chem. Tech Biotechnol. 31 , 135 (1981)). Die Isolierung des gewünschten Produktes ist allerdings u. a. wegen des hohen Siedepunktes des Lösungsmittel schwierig und erfordert eine mehrstufige Extraktion.

In der DE-OS 33 09 564 wird deshalb zur Isolierung des HMF aus Dimethylsulfoxid-enthaltenden Lösungen eine Derivatisierung zu 5-Acetoxymethylfurfural vorgeschlagen. Dies erfordert neben einer Vakuumdestillation außerdem zwei Reaktionsschritte (Acetylierung, Entacetylierung) und somit Zeit- und Chemikalienaufwand.

Mehrere Verfahren verwenden als Reaktionsmedium Gemische von Wasser und organischen

Lösungsmitteln. In der US-PS 2 929 823 wird Furfural zu wäßrigen Saccharidlösungen zugegeben und kurzzeitig (0,1 - 120 sec) auf 250 - 380 °C erhitzt. Teerartige Nebenprodukte werden durch das zugegebene organische Lösungsmittel gelöst, ebenso wie HMF. Die Reindarstellung des HMF erscheint dadurch nur schwierig durchführbar.

Ein weiteres zweiphasiges Verfahren ist in der DE-OS 30 33 527 beschrieben. Dabei werden unter milderen Bedingungen (unter 100 °C) fructosehaltige wäßrige Lösungen mit sauren Kationenaustauschern zersetzt, wobei ein organisches Lösungsmittel, das nicht mit Wasser mischbar ist, aber dennoch ein gutes Lösungsvermögen für HMF besitzt, zugegen ist. Der große Nachteil dieses Verfahrens besteht darin, daß ein sehr großer Überschuß des organischen Lösungsmittels, bezogen auf die wäßrige Phase, (> 7:1) notwendig ist und die erforderlichen Lösungsmittel teuer und giftig sind. Außerdem macht die sehr gute Löslichkeit von HMF in Wasser jegliche Extraktion des Produktes mit organischen Lösungsmitteln aus wäßrigen Lösungen außerordentlich schwierig.

In der Veröffentlichung C. Fayet et al, Carbohydr. Res. 122 , 59 (1983), wird die Zersetzung von Sacchariden ohne Lösungsmittel, jedoch mit äquimolaren Katalysatormengen beschrieben. Der Katalysator Pyridinhydrochlorid ist jedoch für eine technische Anwendung des Verfahrens ungeeignet. Zudem schließt sich nach Zugabe von Wasser eine langwierige Extraktion (20 h) mit Ethylacetat an.

Wie aus den obigen Beispielen hervorgeht, ist das größte Problem bei der Herstellung von HMF, besonders in größerem Maßstab, dieses Produkt von Ausgangsstoffen, Nebenprodukten und Lösungsmitteln abzutrennen. Dies kommt auch in der Veröffentlichung von D. W. Brown et al, J. Chem. Tech. Biotechnol. 32 , 920 (1982) zum Ausdruck, wo es sinngemäß heißt, daß die neueren Methoden der HMF-Herstellung den Nachteil hätten, daß das Produkt in wäßriger Phase oder in einem polaren Lösungsmittel vorliegt, woraus die Isolierung schwierig sei.

Es war deshalb überraschend, daß gerade das Vorliegen des HMF in wäßriger Lösung einen außergewöhnlichen Vorteil bietet und es möglich ist, bei der Auferbeitung der Reaktiousmischung unter ausschließlicher Verwendung von Wasser als Elutionsmittel HMF in großer Reinheit und in guter Ausbeute herzustellen.

Die Verwendung von teueren und gefährlichen organischen Lösungsmitteln, langwierige Extraktionsverfahren mit schwierigen Phasentrennungen, Hochvakuumdestillationen mit der Gefahr der Zersetzung entfallen völlig, da im erfindungsgemäßen Verfahren sowohl die Reaktion in Wasser, insbesondere aber die Aufarbeitung (Trennung) und die Kristallisation des HMF aus Wasser vorgenommen werden können. Dabei soll das erfindungsgemäße Verfahren solche Verfahren nicht ausschließen, bei denen zwar die Reaktion ggf. auch unter Zusatz von organischen Lösungsmitteln zu Saccharid-/HMF- Gemischen führt, nach Entfernung des organischen Lösungsmittels jedoch eine Isolierung des HMFs in einer Chromatographie mit Wasser als Elutionsmittel erfolgt.

Aus der Literaturstelle G. Bonn, Journal of Chromatography, 322 (1985) 411-424, ist zwar schon die analytische Auftrennung verschiedener Zucker und Zuckerabbauprodukte mit Hilfe von HPLC (H igh P erformance L iquid C hromatography oder H igh P ressure L iquid C hromatography) beschrieben worden. Indessen erfordert diese Arbeitsweise die Verwendung besonders kleiner Korngrößen für das Trennmaterial (z.B. 8 bzw. 25 ym) und einen sehr hohen Druck (ca. 100 bar) zum Durchströmen der mobilen Phase durch die stationäre Phase. Hinzu kommt der sehr hohe Kostenaufwand für diesen speziellen Ionenaustauscher. Dies alles hat zur Folge, daß die Verwendung der HPLC außerhalb der Analytik lediglich für einige sehr wertvolle Produkte in Frage kommt. Im vorliegenden Fall gelingt es dagegen, unter Verwendung von handelsüblichen Ionenaustauschern und im dafür üblichen Druckbereich selbst in technischen Dimensionen die angestrebte Trennung erfolgreich durchzuführen.

Wenn man bedenkt, wie komplex eine mehrfache Wasserabspaltung aus einer Hexose verläuft, und daß sowohl aus Fructose wie auch aus dem Reaktionsprodukt HMF, sogenannte Huminstoffe, teils löslich, teils unlöslich entstehen, so ist es überraschend, daß HMF in so großer Reinheit bei der angewandten Chromatographie anfällt.

Überraschend war außerdem die Menge an Reaktionsprodukt, die pro Harzvolumen auf der Chromatographiesäule getrennt werden kann. Während die Menge an Monosacchariden häufig ca. 2% des Bettvolumens beträgt, läßt sich bei Fructose-/HMF-Gemischen eine bis zu doppelt so hohe Trockensubstanzmenge, bezogen auf Harzbettvolumen, vollständig, d. h. ohne Mischfraktion trennen.

Überraschend einfach ist bei dem erfindungsgemäßen Verfahren die Abtrennbarkeit der unlöslichen Nebenprodukte. Als Nebenprodukte der Dehydratisierung von Kohlenhydraten entstehen, in der Literatur "Huminstoffe" genannte Polymerisate. Diese können in unterschiedlicher Form anfallen, so z. B. als Teere (US-PS 2 929 823), als feines Pulver, oder wie im vorliegenden erfindungsgemäßen Verfahren bei Verwendung von Fructoselösungen und Oxalsäure oder Schwefelsäure als Katalysator, als Perlpolymerisate (hier Poly-HMF genannt).

Diese Feststoffe mit der Elementaranalyse von ungefähr C = 63%, H = 4%, O = 33% sind

wegen ihrer Korngröße von ca. 1 mm

Durchmesser sehr leicht abzufiltrieren und besitzen interessante Eigenschaften. Sie sind deshalb ein wertvolles Nebenprodukt des erfindungsgemäßen Verfahrens.

Die Ausgangslösung, z. B. ein Fructosesirup mit 89% Fructose in der Trockensubstanz (Rest: Glucose 9% und Oligosaccharide), wird als 25 - 50%ige Lösung in einen Autoklav eingebracht und mit der Katalysatorsäure auf pH 1,8 gebracht. Die Mischung wird unter Rühren (Drehzahl 120 UpM) auf eine Temperatur zwischen 120 und 150 °C erhitzt und ca. 2 Stunden bei dieser Temperatur gehalten. Anschließend wird abgekühlt.

Wenn das als Nebenprodukt anfallende Perlpolymerisat (Poly-HMF) gewonnen werden soll, wird dies abfiltriert, sonst wird die Reaktionslösung mit einer Base neutralisiert und der Niederschlag gemeinsam abfiltriert. Dabei ist es vorteilhaft, wenn die zugeführten Kationen die gleichen sind, die auch in der Ionenaustauschersäule der anschliessenden Chromatographie als Gegenionen vorliegen, d. h. man neutralisiert mit Calciumhydroxyd oder mit Calciumcarbonat, wenn die zur Chromatographie benutzte Form des Austauscherharzes die $Ca^{2+}$-Form ist (z. B. Lewatit ® TSW 40, $Ca^{2+}$ - Form, als stark saurer gelförmiger Kationenaustauscher auf Polystyrolsulfonsäurebasis mit ca. 4% Divinylbenzol vernetzt). Andere Harze werden durch das erfindungsgemäße Verfahren nicht ausgeschlossen.

Falls die Reaktionslösung nicht neutralisiert wird, kann sie auch nach Filtration direkt über eine Ionenaustauschersäule in der $H^+$-Form chromatographiert werden.

In diesem Fall werden Trennungen erreicht mit so verschiedenen Harzen, wie z. B. starksauren, hochvernetzten, makroporösen Kationenaustauschern auf Polystyrolsulfonsäurebasis (z. B. Lewatit ® SP 112), schwachsauren, makroporösen Kationenaustauschern auf Polyacrylatbasis (z. B. Lewatit ® CMP LF) oder schwach vernetzten, gelartigen Kationenaustauscherharzen (z. B. Lewatit ® TSW 40). Auf allen Harzen werden gefärbte und saure Nebenprodukte von HMF abgetrennt, das nach den Sacchariden (Fructose) eluiert wird. Die zu trennende Menge der Reaktionsmischung liegt im Bereich von 10 % des Harzbettvolumens (Aufgabelösung mit ca. 35 % TS).

Die filtrierte Lösung wird vorteilhaft direkt, also ohne Konzentrierung oder Verdünnung, als Aufgabelösung der Chromatographie verwendet. Dies hat insbesondere verfahrenstechnische Vorteile, wenn mit größeren Mengen gearbeitet wird. Bei der Chromatographie ist es möglich, bis zu 20% des Harzvolumens als Aufgabevolumen, z. B. als ca. 30%ige Lösung, zu verwenden. Als Elutionsmittel wird Wasser verwendet mit einer linearen Durchflußgeschwindigkeit von verzugsweise 2.5 bis 3.5cm/min, insbesondere von ca. 3,2 cm/min im Harbett. Die Trennung erfolgt bei einer Temperatur zwischen 35 und 95 °C und eine Temperatur von ca. 55 - 85 °C ist für die Trennung vorteilhaft. Je nach der Aufgabemenge kann man die Auftrennung in HMF-und Zuckerfraktion ohne oder mit einer Mischfraktion erhalten. Bei einer Aufgabemenge von ca. 8% des Bettvolumens (33,2% TS) läßt sich das Eluat auftrennen in eine Vorfraktion, die einen Teil der Salze, Farbe und Glucose enthält.

Die erste Produktfraktion enthält die Hauptmenge Fructose, die folgende zweite Produktfraktion enthält HMF, wobei in dieser Fraktion praktisch keine Salze gefunden werden. Beim Einengen dieser Fraktion erhält man einen bernsteinfarbigen Sirup, der in kurzer Zeit bei Temperaturen unter 20 °C kristallisiert mit Reinheiten 90%. Diese Reinheit ist für viele Anwendungsfälle ausreichend.

Dieser Sirup kann entweder durch Kühlungskristallisation oder durch isotherme Kristallisation unter 20 °C bei gleichzeitiger Verminderung des Restwassergehaltes durch Vakuumverdampfung weiter gereinigt werden. In beiden Fällen erhält man analysenreines HMF.

Die hauptsächlich Fructose enthaltende Fraktion kann nach Einengen einer erneuten Dehydratisierung im Autoklav zugeführt oder als Verdünnungslösung höher konzentrierter Saccharidgemische verwendet werden.

Eine bei größerer Aufgabemenge erhältliche Mischfraktion kann rechromatographiert oder als solche verwendet werden. In Fig. 1 ist ein Fließschema der HMF-Herstellung dargestellt; in Fig. 2 wird ein Elutionschromatogramm gezeigt (Beispiel 5)

Der nach der Chromatographie anfallende auf einen Restwasser-gehalt < 20% Wasser eingedampfte HMF-haltige Sirup - wobei das Eindampfen im Vakuum bei einer Temperatur < 80 °C erfolgt - wird einer Kühlungskristallisation unterworfen; dabei wird der Sirup in einer geeigneten rühr- und kühlbaren Kristallisationsapparatur zuerst schnell auf 20 °C abgekühlt. Die weitere Abkühlung erfolgt mit einer Kühlrate von 2 - 20 °/h, vorwiegend im Bereich von 2 - 5 °/h. Bei der in Abhängigkeit vom Wassergehalt sich ergebenden Übersättigungstemperatur, z. B. 10 °C, wird die Masse mit HMF-Kristallen angeimpft und die Kühlung bis auf eine Temperatur weitergeführt, bei der ein Kristallgehalt im Magma von 55 - 60% erreicht ist.

Bei dieser Temperatur werden die Kristalle von der Mutterlauge, z. B. in einer Siebkorbzentrifuge oder einer Drucknutsche abgetrennt. Die so gewonnenen Kristalle haben eine Reinheit > 97%.

Die beim Trennvorgang anfallende Mutterlauge wird einer zweiten Kristallisation unterworfen und, wie oben beschrieben, Kristalle und Mutterlauge

getrennt. Der Muttersirup dieser zweiten Kristallisation wird rechromatographiert.

Die Kristalle der ersten und zweiten Kristallisation werden durch Temperaturerhöhung aufgeschmolzen, filtriert und wie oben beschrieben, kristallisiert. Dabei wird analysenreines HMF (Reinheit > 99%) erhalten.

Die Aufgabemenge bei der Chromatographie kann besonders hoch gewählt werden, wenn die Chromatographieanlage aus mehreren Säulen besteht. Dies ist nicht nur aus räumlichen Gründen bei Großanlagen ein Vorteil, vielmehr gestattet eine Mehrsäulenanlage, einen Teil des Elutionsstromes an einer geeigneten Stelle auszuleiten und nur den Rest die gesamte Anlage durchlaufen zu lassen.
Bei dem hier dargestellten erfindungsgemäßen Verfahren wurde eine Dreisäulenanlage verwendet, wobei die drei Säulen mit gleichem Harzbettinhalt in Reihe hintereinander durchströmt werden.

Die Elution über die ersten beiden Säulen wird so lange in einen separaten Behälter geleitet, bis im Auslauf nach Säule 2 refraktometrisch das Trockensubstanz-Maximum überschritten ist. Erst dann wird der Elutionsstrom über ein Ventil auf die dritte Säule geleitet und die Chromatographie in gewohnter Weise fortgesetzt (fraktioniert).

Über eine Dreisäulenanlage (Gesamtlänge: 10 m; Durchmesser: 25 cm), die z. B. mit dem vorgenannten Kationenaustauscher TSW 40 in der $Ca^{++}$-Form gefüllt ist, lassen sich beispielsweise 120 kg Reaktionslösung (7,8% HMF) so trennen, daß nur HMF-haltige Lösungen auf die dritte Säule geleitet werden und auf dieser zum größten Teil vollständig von Fructose getrennt werden.

Beispiel 1

11 kg Fructose werden in 33 1 Wasser gelöst und 110 g Oxalsäuredihydrat zugegeben. Der Ansatz wird in einem Rührautoklav (Drehzahl 120 UpM) innerhalb von 15 min auf 140°C erwärmt und nach 130 min bei einer Temperatur von 135 - 142°C wieder auf 40°C gekühlt. Der entstandene Feststoff (Poly-HMF) wird in einer Druckfilternutsche abfiltriert (1,3 kg; nach der Elementaranalyse entspricht dies einer Dehydratisierung von 2,05 kg Fructose) und das Filtrat mit Calciumcarbonat neutralisiert (pH 5) und filtriert. Das Filtrat (41,0 kg) enthält 6,3% HMF, das entspricht 2,58 kg HMF, die aus 3,7 kg Fructose entstanden sind. Bezogen auf eingesetzte Fructose beträgt die Ausbeute 33,6%. Die Lösung enthält noch 4,3 kg nicht umgesetzte Fructose. Die Ausbeute, bezogen auf insgesamt umgesetzte Fructose, beträgt demnach 55%. Neben HMF und Fructose sind noch geringe Mengen Glucose und Oligosaccharide und nach Entfernung des unlöslichen Poly-HMF auch lösliche, hochmolekulare Huminstoffe enthalten, die sich jedoch bei der anschließenden Chromatographie vollständig von HMF abtrennen lassen.

Beispiel 2

20 kg Zichorienwurzeln mit einem Inulingehalt von 17,8% (gemessen als Fructose nach enzymatischer Hydrolyse) werden zu einem Mus zerkleinert. Dieses wird mit 20 kg Wasser gemischt und mit Schwefelsäure der pH-Wert auf 1,8 eingestellt (1,3 kg 20%ige Schwefelsäure). Das angesäuerte Mus wird in einem Rührautoklav innerhalb von 15 min auf 140°C erhitzt und 2 h bei dieser Temperatur gehalten (Druck 7,2 bar), dann auf 70°C gekühlt und der Inhalt in einer Drucknutsche filtriert. Neben einem Rückstand von 7,5 kg erhält man eine Lösung (32 kg) mit folgender Analyse, bezogen auf Trockensubstanzgehalt: HMF = 16,6%, Fructose = 59,5%, Glucose = 7,2%, Rest = 16,7%. Dies entspricht folgenden Mengen: 298 g HMF (entsprechend 426 g umgewandelter Fructose), 1062 g Fructose, 131 g Glucose und 304 g Restsaccharide.
Bezogen auf die berechnete Menge Inulin in 20 kg Zichorienwurzeln, das sind 3,3 kg, beträgt die HMF-Ausbeute 13%, die isolierbare Menge an Fructose 30%. Das Filtrat wird mit 380 g Calciumcarbonat auf pH 6,5 gebracht und nochmals filtriert.

Beispiel 3

Geschnitzelte Zichorienrüben werden in einer Gegenstromextraktionsapparatur (DDS-Extraktor) mit Wasser von 70°C im Gegenstrom extrahiert, wobei man einen Rohextrakt mit 16% Inulin erhält, der auf 30% TS-Gehalt eingeengt wird. Die Ausbeute an Inulin, bezogen auf die lösliche Trochensubstanz in den Pflanzenteilen beträgt 86,8%. Die Zersetzung im Autoklaven erfolgt dann analog Beispiel 1.

Beispiel 4

Zerkleinerte Zichorienwurzeln werden mit 20%iger Schwefelsäure (Menge: 7 ml konz. Schwefelsäure/kg Zichorienmus) gleichmäßig gemischt und 2,5 Stunden bei einer Temperatur von 80°C gehalten. Nach dem Prinzip des Henze-Dämpfers wird die Masse in einer vorerwärmten Drucknutsche weiter aufgeschlossen, indem sie mit Dampf von 6 bar Druck 1 Stunde lang beaufschlagt wird. Danach wird entspannt und die verflüssigte Masse direkt oder nach Filtration in einen Autoklaven überführt und nach Beispiel 2 umgesetzt.

Beispiel 5

903 g eines wäßrigen Kohlenhydrat/HMF-Gemisches mit 7,8% HMF und einem Brechungsindex von 1.387, wie es bei einer Autoklavenreaktion (90% Fructose, 10% Glucose + Restsaccharide, bezogen auf Trockensubstanzgehalt) entstanden ist, nachdem mit Calciumhydroxyd auf pH 6,7 neutralisiert und filtriert wurde, werden auf eine 10 l-Säule aufgegeben. In der Säule (Durchmesser 8 cm, Länge 200 cm) befindet sich das Trennharz Lewatit TSW 40 der Fa. Bayer in der $Ca^{2+}$-Form. Die Säule und das Elutionswasser werden auf 65°C temperiert; der Durchfluß beträgt 10 1/h. Es ergibt sich das Elutionsdiagramm der Fig. 2.

Die Zuckerfraktion enthält 152,4 g an Zuckern, davon 87% als Fructose und 13% als Glucose. Die HMF-Fraktion ergibt nach Einengen im Vakuum 55 g mit einer Reinheit von 95%. Das sind 78% des in der Aufgabelösung vorhandenen HMFs.

Beispiel 6

1598 g einer mit Ionenaustauscher entsäuerten Lösung mit dem Brechungsindex 1,3834 und einem HMF-Gehalt von 3,9% werden über eine 10 l-Säule chromatographiert, die Lewatit TSW 40 in der $Na^{+}$-Form enthält (Durchmesser 8 cm, Höhe 200 cm), Temperatur 65°C, Durchfluß 10 l/h. Die nur HMF-enthaltenden Fraktionen werden eingeengt und ergeben 50,0 g HMF in kristalliner Form. Bezogen auf das in der Aufgabenlösung vorhandene HMF werden 80% so gewonnen.

Beispiel 7

Die HMF-Fraktionen zweier, großtechnisch in einer Dreisäulenanlage mit 13 $m^3$ Inhalt und einer Füllung aus dem Polystyrolsulfonsäure-Kationenaustauscher in $Ca^{2+}$-Form durchgeführter Chromatographien (Fraktion a: 143 kg TS-Gehalt, davon 137 kg HMF, 1,0 kg Fructose, 4,6 kg Restsaccharide; Fraktion b: 176 kg TS-Gehalt, davon 163 kg HMF, 3,0 kg Fructose, 1,9 kg Glucose und 7,7 kg Restsaccharide) werden unter 80°C im Vakuum eingedampft bis zu einem Restwassergehalt von ca. 7%. In einem kühl- und rührbaren Kristallisationsgefäß wird mehrstufig kristallisiert. Bei der 1. Kühlungskristallisation wird das Substanzgemisch von 20°C mit einer Kühlrate von 5°/h abgekühlt, bei 10°C mit HMF-Kristallen geimpft und die Masse weiter auf 4°C gekühlt.
Man erhält einen Kristallbrei, der mit einer Siebkorbzentrifuge in 124,5 kg Kristalle und 210,9 kg Mutterlauge getrennt wird (Kristallausbeute 39%).
Die Kristalle haben eine Reinheit von 97% und sind gelb gefärbt.
Die Mutterlauge enthält, bezogen auf TS-Gehalt, 88,4% HMF und 11,6% Restsaccharide und insgesamt 17% Wasser. Der Wassergehalt läßt sich mit der KF-Titration mit Hydranal Composite 5K für Ketone und Hydranal-Arbeitsmedium K für Ketone der Fa. Riedel-de Haen bestimmen. Diese Mutterlauge wird nach Eindampfen auf einen Endwassergehalt von 3% einer zweiten Kristallisation unterworfen und in 99 kg Kristalle und 111 kg Mutterlauge in einer Siebkorbzentrifuge getrennt. Die Mutterlauge wird rechromatographiert.

Beispiel 8

124 kg kristallisiertes HMF (97%ig) wird durch leichtes Erwärmen geschmolzen und über eine Druckfilternutsche filtriert, wobei 1 kg unlöslicher Feststoff abgetrennt wird. Anschließend wird der Sirup in einem kühl- und rührbaren Kristallisationsgefäß wie in Beispiel 7 beschrieben, auf +5°C abgekühlt, das Magma zentrifugiert, und dabei werden 45 kg Kristalle erhalten; die Mutterlauge und das Waschwasser werden erneut, wie oben beschrieben, auf 0°C abgekühlt, das Magma zentrifugiert und dabei 25,5 kg Kristalle erhalten. Die Mutterlauge wird in einer dritten Kristallisationsstufe, wie oben beschrieben, auf -5°C abgekühlt und das Magma zentrifugiert. Dabei erhält man 12,2 kg Kristalle. Insgesamt ergibt sich eine Menge von 82,7 kg kristallines HMF mit der Reinheit von 99,4%. Die Kristallausbeute beträgt 67%. Die Kristalle sind schwach gelb gefärbt.
Es fallen insgesamt 50 kg Mutterlauge an, die nach Beispiel 7 kristallisiert werden.

Beispiel 9

Anstelle einer Kühlungskristallisation kann man die aus der Chromatographie analog Beispiel 7 anfallenden HMF-Fraktionen auf einen Restwassergehalt von 20 - 25% Wasser im Vakuum unterhalb einer Temperatur von 80°C eindampfen und in einem rühr- und kühlbaren Kristallisationsgefäß auf 10°C abkühlen. Unter Rühren und Temperaturkonstanthaltung wird vorsichtig ein Vakuum angelegt, das zwischen 5 und 100 mbar regelbar ist. Bei einem Wassergehalt von 10% saugt man Impfkristalle in das Kristallisationsgefäß ein und trennt Kristalle und Mutterlauge analog Beispiel 7, wenn das Magma ungefähr einen Kristallgehalt von 50% hat.

**Ansprüche**

**1.** Verfahren zur Herstellung von 5-Hydroxymethylfurfural, wobei Saccharide in wäßriger Lösung, ggf. unter Zusatz von organischen Lösungsmitteln, über 100 °C mit einem sauren Katalysator zu einem Gemisch von Hexosen und HMF zersetzt werden, dadurch gekennzeichnet, daß diese Reaktionsmischung nach Entfernung von ggf. vorhandenen organischen Lösungsmitteln und den als Nebenprodukt angefallenen polymerem Feststoffen unter ausschließlicher Verwendung von Wasser als Elutionsmittel über Ionenaustauschersäulen bei einer Temperatur zwischen 35 und 95 °C so getrennt wird, daß die abgetrennten Saccharidfraktionen erneut verwendet werden, die HMF-Fraktion ggf. zur Kristallisation gebracht wird und abgetrennte Misch- oder Zwischenfraktionen rechromatographiert werden.

**2.** Verfahren nach Anspruch 1, dadurch gekennzeichnet, daß die zu zersetzende Lösung eine wäßrige Lösung von Fructose bzw. einer Fructose enthaltenden Mischung oder eines Fructose enthaltenden Oligo- oder Polysaccharids oder Naturstoffes, z. B. suspendierten Pflanzenteilen, die derartige Kohlenhydrate enthalten, ist.

**3.** Verfahren nach Anspruch 2, dadurch gekennzeichnet, daß die Hexosen enthaltende wäßrige Lösung in situ durch die Spaltung eines Oligo- oder Polysaccharides, wie z. B. Saccharose oder Inulin oder derartige Kohlenhydrate enthaltende pflanzliche Rohstoffe, z. B. Zichorienwurzeln oder Topinamlurknollen, hergestellt wird.

**4.** Verfahren nach Anspruch 2, dadurch gekennzeichnet, daß die Saccharidkonzentration zwischen 10 und 50% TS-Gehalt, vorzugsweise 30 - 40% TS, beträgt, insbesondere aber gleich oder größer ist, wie die Konzentration der Aufgabelösung bei der sich anschließenden Chromatographie.

**5.** Verfahren nach Anspruch 1, dadurch gekennzeichnet, daß die Katalysatorsäure, z. B. Oxalsäure oder Schwefelsäure, in einer Konzentration zwischen 0,01 und 5% zugesetzt wird und möglichst ein schwer lösliches Salz mit der zugesetzten Base bildet, z. B. Calciumoxalat oder Calciumsulfat, falls die Reaktionslösung neutralisiert wird.

**6.** Verfahren nach Anspruch l, dadurch gekennzeichnet, daß die Reaktionsmischungen nach geeigneter Vorbehandlung, z. B. Neutralisation, Filtration, über Kationenaustauschersäulen chromatographiert werden, wobei die Kationenaustauscherharze in 1-, 2-,
oder 3-wertiger Salzform vorliegen oder wenn die Reaktionslösung nicht neutralisiert wird, die Ionenaustauscher in der $H^+$-Form verwendet werden.

**7.** Verfahren nach Anspruch 6, dadurch gekennzeichnet, daß die Chromatographie bei Temperaturen zwischen 55 und 85 °C durchgeführt wird.

**8.** Verfahren nach Anspruch 6, dadurch gekennzeichnet, daß das Aufgabevolumen bei der Chromatographie 5 bis 20% des Harzvolumens beträgt, wobei die Konzentration der aufgegebenen Lösung, gemessen als Brechzahl zwischen 1.36 bis 1.45, vorzugsweise zwischen 1.37 bis 1.39, liegt.

**9.** Verfahren nach den Ansprüchen 6 bis 8, dadurch gekennzeichnet, daß mit linearen Strömungsgeschwindigkeiten im Harzbett von 2,0 bis 5,0 cm/min, vorzugsweise 2,5 bis 3,5 cm/min, gearbeitet wird.

**10.** Verfahren nach Anspruch 6, dadurch gekennzeichnet, daß die Saccharide enthaltenden Fraktionen, eine HMF enthaltende Fraktion und ggf. die Mischfraktionen getrennt gesammelt werden, wobei die Saccharide enthaltenden Fraktionen wieder einer Reaktion nach Anspruch 1 zugeführt werden, die HMF enthaltenden Fraktionen zur Kristallisation gebracht oder als solche verwendet werden und die sowohl Saccharide als auch HMF enthaltenden Mischfraktionen erneut nach Anspruch 6 chromatographiert oder als solche verwendet werden.

**11.** Verfahren nach Anspruch 6, dadurch gekennzeichnet, daß die Chromatographie über 2 oder mehrere in Reihe geschaltete Säulen so erfolgt, daß die vorwiegend Saccharide enthaltenden Lösungen nicht über sämtliche Säulen geleitet werden, sondern z. B. bei einer 3-Säulen-Anlage schon nach der 2. Säule ausgeschleust werden.

**12.** Verfahren nach den Ansprüchen 1 und 6, dadurch gekennzeichnet, daß die wäßrige HMF enthaltende Lösung im Vakuum eingeengt und bei Temperaturen zwischen -5 und +30 °C, bevorzugt zwischen 0 und 10 °C, einer Kühlungskristallisation oder unter 30 °C einer Verdampfungskristallisation unterworfen wird.

**13.** Verfahren nach Anspruch 12, dadurch gekennzeichnet, daß der Wassergehalt in der HMF-Lösung maximal 20% beträgt, vorzugsweise jedoch um den Bereich 5 - 10% liegt, die Kühlungskristallisation unter Rührung bei + 30 ° C begonnen und mit einer Kühlrate von 2 - 20 ° /h bis auf eine Temperatur von -5 bis +10 ° C durchgeführt wird, wobei ein Kristallgehalt im fertigen Magma von ca. 50% resultiert, und das Magma anschließend auf einer Siebkorbzentrifuge oder auf einer Drucknutsche in Sirup und Kristalle getrennt wird, wobei die erhaltenen HMF-Kristalle eine Reinheit von etwa 97% haben.

**14.** Verfahren nach Anspruch 13, dadurch gekennzeichnet, daß der erhaltene Muttersiurp einer zweiten Kühlungkristallisation unterworfen wird.

**15.** Verfahren nach den Ansprüchen 13 und 14, dadurch gekennzeichnet, daß die erhaltenen HMF-Kristalle durch Temperaerhöhung wieder aufgeschmolzen werden und nach Wasserzusatz einer Umkristallisation gem. den Ansprüchen 12 bis 14 unterworfen werden, wobei die umkristallisierten Kristalle eine Reinheit von 99% an HMF haben.

## Claims

**1.** Process for preparing 5-hydroxymethylfurfuraldehyde, wherein saccharides in aqueous solution, eventually with added organic solvents, above 100 $^0$C are decomposed with an acid catalyst leading to a mixture of hexoses and HMF, wherein this reaction mixture, if present, after removal of organic solvents, and the polymer solids yielded as a by-product, is separated with exclusive utilization of water as elution agent via ion exchange columns, at a temperature between 35 and 95 ° C, in such a way, that the separated saccharide fractions are used repeatedly, the HMF-fraction, if desired, is crystallized, and separated mixed or intermediate fractions are rechromatographed.

**2.** Process according to claim 1, wherein the solution to decompose is an aqueous solution of fructose resp. a solution of a fructose-containing mixture or a fructose-containing oligo- or polysaccharide or a natural substance, e.g. suspended parts of plants, which contain such carbohydrates.

**3.** Process according to claim 2, wherein the hexose-containing aqueous solution is prepared in situ by splitting oligo- or polysaccharides, like sucrose or inulin, or vegetable raw material, which contains such carbohydrates, e.g. chicory roots or tubers of the Jerusalem artichoke.

**4.** Process according to claim 2, wherein the concentration of sacharides is between 10 and 50 % d.s., preferably 30 to 40 % d.s., but, however, equal or higher than the concentration of the feeding solution in the following chromatography.

**5.** Process according to claim 1, wherein the acid catalyst e.g. oxalic acid or sulphuric acid, is used in a concentration between 0,01 and 5 % and forms salts of low solubility with the added base, e.g. calcium oxalate or calcium sulphate, if the reaction mixture is neutralized.

**6.** Process according to claim 1, wherein the reaction mixture after appropriate preliminary treatment, e.g. neutralization, filtration, is chromatographed on cation exchanger colums, where the exchange resins are used in 1-, 2- or 3-valent salt form, or, if the reaction mixture is not neutralized, the ion exchangers are used in the $H^+$-form.

**7.** Process according to claim 6, wherein the chromatography is realized between 55 and 85 $^0$C.

**8.** Process according to claim 6, wherein the amount of feeds syrup for the chromatography is 5 to 20 % of the volume of the resin bed and the concentration of the feeds, measured as refractive index, is between 1,36 and 1,45, preferably between 1,37 and 1,39.

**9.** Process according to claim 6 - 8, wherein the chromatographic separation is performed with linear flow-rates in the resin bed between 2,0 and 5,0 cm/min, preferably 2,5 and 3,5 cm/min.

**10.** Process according to claim 6, wherein saccharidecontaining fractions, a fraction containing HMF and potentially obtained mixed fractions are collected separately, and the saccharide-containing fractions are reacted according claim 1, the HMF containing fractions are crystallized, or used as such, and the mixed fractions, containing both, saccharides and HMF, are separated according to claim 6 or used as such.

**11.** Process according to claim 6, wherein the chromatographic separation is performed on 2 or more columns, which are connected in series, in such a way, that the predominantly saccharides-containing solutions are not led over all colums, but, e.g. in a three-column-equipment, are let out after the 2. column.

**12.** Process according to claims 1 and 6, wherein the aqueous HMF-containing solution is concentrated in vacuum and subjected, at temperatures between - 5° and + 30°C, preferably between 0° and 10° C, to a crystallization by cooling or, below 30° C, to a crystallization by evaporation.

**13.** Process according to claim 12, wherein the water-content in the HMF-solution is maximally 20 %, preferably however, in the range 5 - 10 %, the crystallization by cooling under stirring is begun at + 30 °C, and accomplished to a temperature of - 5 to + 10 °C with a colling rate of 2 - 20 °/h, resulting in a crystal content of app. 50 % in the final magma, and the magma is separated subsequently with a filter centrifuge or a pressure nutsche filter in syrup and crystals, whereby the obtained HMF crystals have a purity of app. 97 %.

**14.** Process according to claim 13, wherein the received mother liquor is subjected to a second crystallization by cooling.

**15.** Process according to claim 13 and 14, wherein the obtained HMF-crystals are liquefied again by rise in temperature, and after addition of water, are subjected to recrystallization according to claims 12 - 14, whereby the recrystallized HMF has a purity of 99 %.

## Revendications

**1.** Procédé de préparation du 5-hydroxyméthylfurfural (HMF) dans lequel des saccharides en solution aqueuse, le cas échéant avec addition de solvants organiques,sont décomposés par chauffage à plus de 100°C avec un catalyseur acide pour former un mélange d'hexoses et de HMF,
**caractérisé** en ce que ce mélange réactionnel, après élimination des solvants organiques, présents le cas échéant, et des solides polymères obtenus comme sous-produits, est séparé, en utilisant exclusivement l'eau comme agent d'élution, dans des colonnes d'échange d'ions à une température entre 35 et 95 °C, de telle manière que les fractions saccharides séparées sont réutilisées, que la fraction HMF est mise à cristalliser, le cas échéant, et que les fractions mélangées ou intermédiaires sont rechromatographiées.

**2.** Procédé selon la revendication 1, caractérisé en ce que la solution à décomposer est une solution aqueuse de fructose ou d'un mélange contenant du fructose, ou d'un oligosaccharide ou d'un polysaccharide contenant du fructose, ou encore de substances naturelles, par exemple des parties de plantes en suspension, contenant de tels hydrates de carbone.

**3.** Procédé selon la revendication 2, caractérisé en ce que la solution aqueuse contenant des hexoses est préparée in situ par dissociation d'un oligosaccharide ou d'un polysaccharide tel que par exemple saccharose ou inuline, ou de matières premières végétales contenant de tels hydrates de carbone, par exemple des racines de chicorée ou des tubercules de topinambour.

**4.** Procédé selon la revendication 2, caractérisé en ce que la concentration en saccharides est comprise entre 10 et 50 % en matière sèche, de préférence de 30 à 40 % en matière sèche, mais qu'en particulier elle est égale ou supérieure à la concentration de la solution mise en oeuvre dans la chromatographie qui fera suite.

**5.** Procédé selon la revendication 1, caractérisé en ce que l'acide catalyseur, par exemple acide oxalique ou acide sulfurique, est ajouté à une concentration entre 0,01 et 5 % et forme, autant que possible, un sel difficilement soluble avec la base ajoutée, par exemple de l'oxalate de calcium ou du sulfate de calcium, au cas où la solution de réaction est neutralisée.

**6.** Procédé selon la revendication 1, caractérisé en ce que les mélanges réactionnels sont chromatographiés au moyen de colonnes d'échange de cations, après un traitement préliminaire approprié, par exemple neutralisation ou filtration, les résines d'échange de cations étant sous la forme de sels monovalents, divalents ou trivalents, ou bien, si la solution de réaction n'est pas neutralisée, les échangeurs d'ions étant alors utilisés sous la forme H+.

**7.** Procédé selon la revendication 6, caractérisé en ce que la chromatographie est réalisée à des températures entre 55 et 85 °C.

**8.** Procédé selon la revendication 6, caractérisé en ce que le volume mis en oeuvre dans la chromatographie est de 5 à 20 % du volume de la résine, la concentration de la solution

mise en oeuvre, mesurée par son indice de réfraction, se situant entre 1,36 et 1,45, de préférence entre 1,37 et 1,39.

**9.** Procédé selon les revendications 6 à 8, caractérisé par le fait de travailler avec des vitesses linéaires de circulation dans le lit de résine comprises entre 2,0 et 5,0 cm/minute, de préférence entre 2,5 et 3,5 cm/minute.

**10.** Procédé selon la revendication 6, caractérisé en ce que les fractions contenant des saccharides, une fraction contenant du HMF et, le cas échéant, les fractions mélangées, sont recueillies séparément, les fractions contenant des saccharides étant à nouveau dirigées vers une réaction selon la revendication 1, les fractions contenant du HMF étant mises à cristalliser ou étant utilisées telle quelles, et les fractions mélangées contenant aussi bien des saccharides que du HMF étant à nouveau chromatographiées selon la revendication 6 ou bien utilisées telles quelles.

**11.** Procédé selon la revendication 6, caractérisé en ce que la chromatographie a lieu sur au moins deux colonnes disposées en série, de telle manière que les solutions contenant surtout des saccharides ne soient pas mises à passer par toutes les colonnes, mais sont, par exemple, dans le cas d'une installation à trois colonnes, déjà éclusées vers l'extérieur après la deuxième colonne.

**12.** Procédé selon les revendications 1 et 6, caractérisé en ce que la solution aqueuse contenant du HMF est concentrée sous vide et soumise à une cristallisation par refroidissement à des températures entre -5°C et +30°C, de préférence entre 0 et 10°C, ou bien à une cristallisation par évaporation à moins de 30°C.

**13.** Procédé selon la revendication 12, caractérisé en ce que la teneur en eau de la solution de HMF est au maximum de 20%, mais de préférence de 5 à 10%, en ce que la cristallisation par refroidissement est commencée sous agitation à 30°C et qu'elle est conduite à une vitesse de refroidissement de 2 à 20°C/heure jusqu'à une température de -5°c à +10°C, d'où il résulte, dans le magma obtenu, une teneur en cristaux d'environ 50%, le magma étant ensuite séparé en sirop et en cristaux au moyen d'une centrifugeuse à cage filtrante ou au moyen d'un filtre-presse, les cristaux de HMF obtenus ayant une pureté d'environ 97%.

**14.** Procédé selon la revendication 13, caractérisé en ce que le sirop-mère obtenu est soumis à une deuxième cristallisation par refroidissement.

**15.** Procédé selon les revendications 13 et 14, caractérisé en ce que les cristaux de HMF obtenus sont à nouveau fondus par augmentation de température et sont soumis, après addition d'eau, à une recristallisation selon les revendications 12 à 14, les cristaux recristallisés ayant une pureté de 99% en HMF.

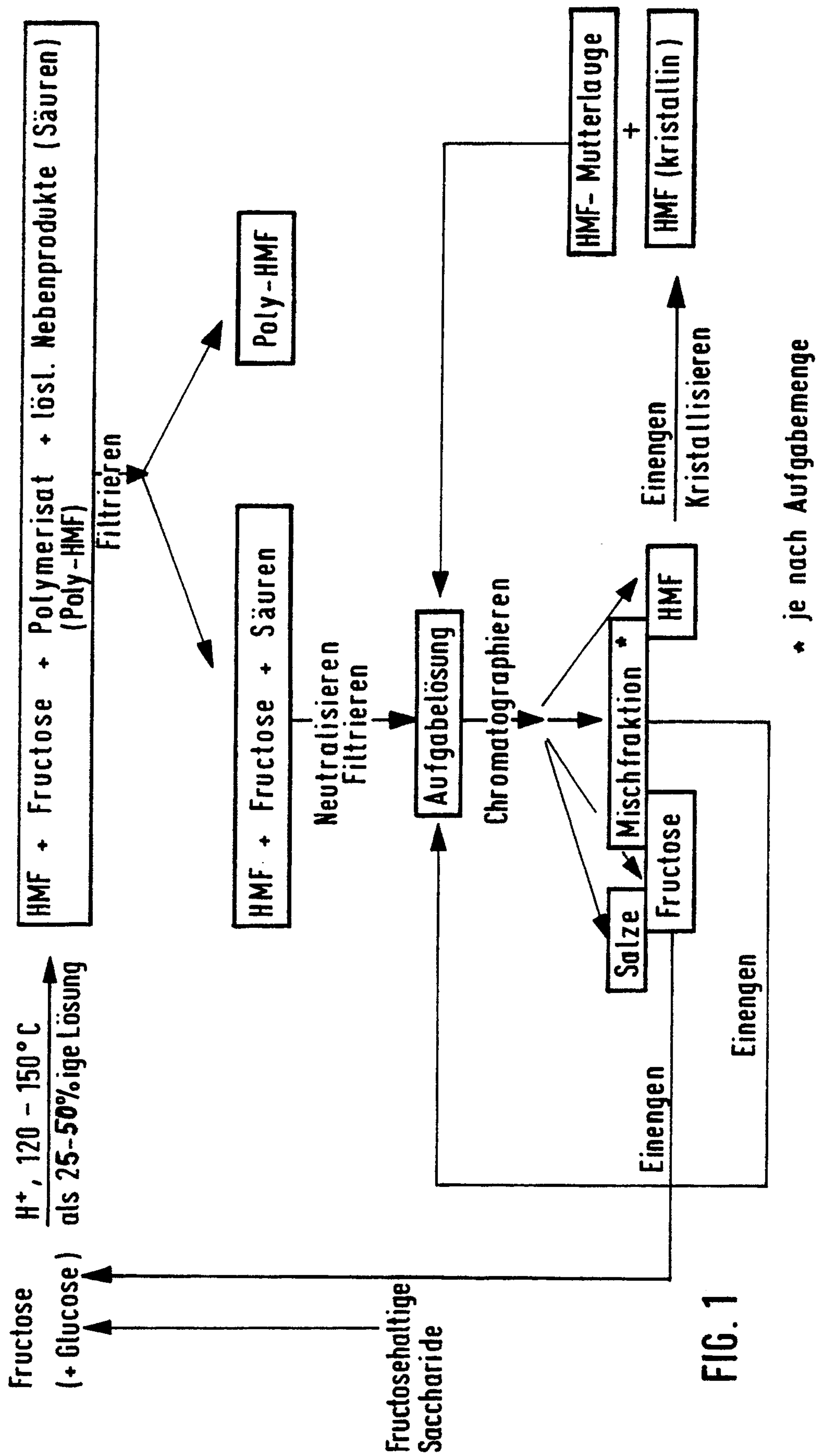
Fructose
(+ Glucose)
Fructosehaltige Saccharide
H+, 120 - 150°C
als 25-50%ige Lösung
HMF + Fructose + Polymerisat + lösl. Nebenprodukte (Säuren)
(Poly-HMF)
Filtrieren
Poly-HMF
HMF + Fructose + Säuren
Neutralisieren
Filtrieren
Aufgabelösung
Chromatographieren
Salze
Fructose
Mischfraktion *
HMF
Einengen
Einengen
Einengen
Kristallisieren
HMF- Mutterlauge
+
HMF (kristallin)
* je nach Aufgabemenge

FIG. 1

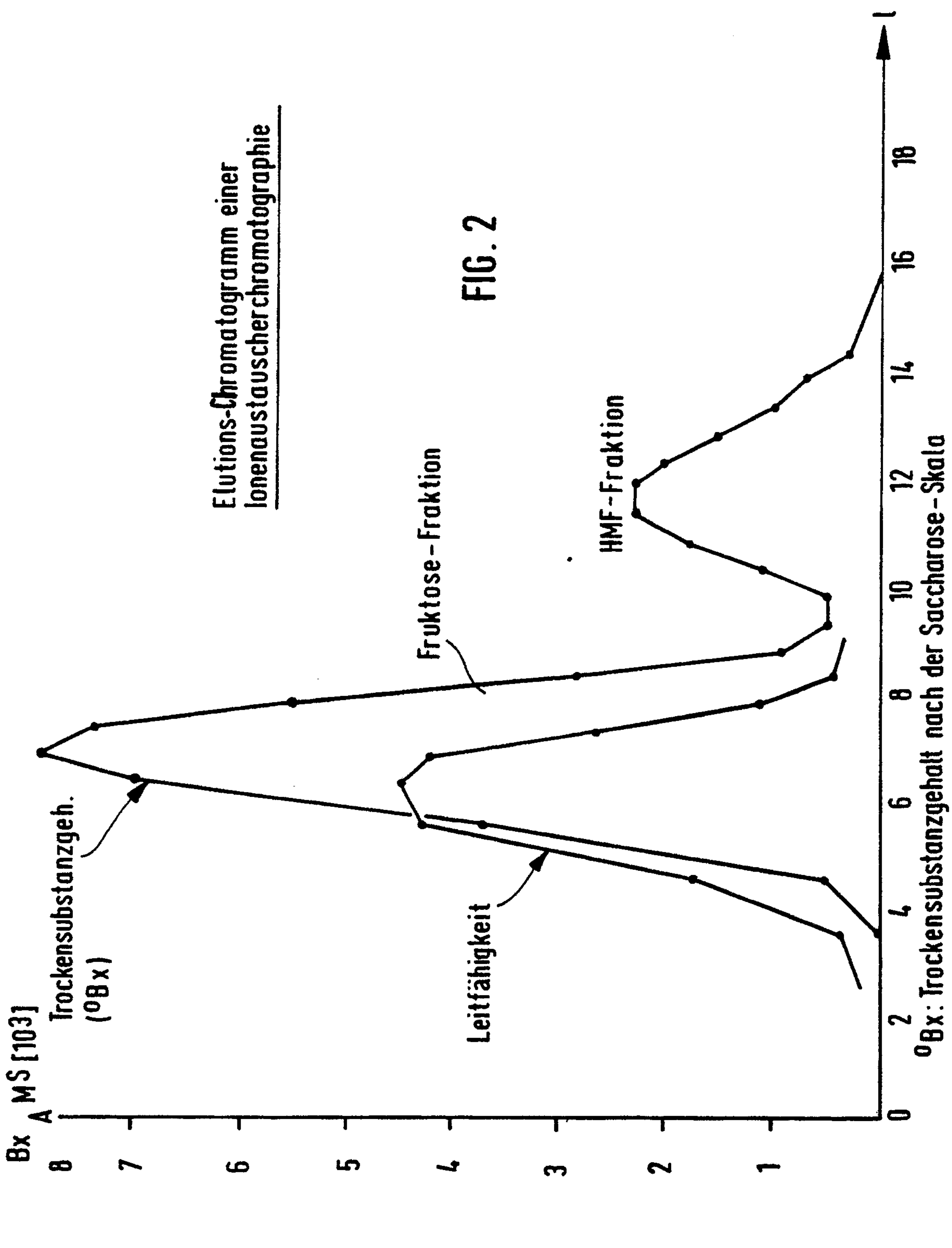
Elutions-Chromatogramm einer Ionenaustauscherchromatographie
Bx
A
MS [10³]
8
7
6
5
4
3
2
1
0
2
4
6
8
10
12
14
16
18
l
Trockensubstanzgeh. (°Bx)
Leitfähigkeit
Fruktose-Fraktion
HMF-Fraktion
°Bx: Trockensubstanzgehalt nach der Saccharose-Skala

FIG. 2